## Europäisches Patentamt

## European Patent Office

## Office européen des brevets

(19)

(11) Veröffentlichungsnummer: **0 246 439**
**B1**

(12) **EUROPÄISCHE PATENTSCHRIFT**

(45) Veröffentlichungstag der Patentschrift:
05.09.90

(21) Anmeldenummer: **87105116.5**

(22) Anmeldetag: **07.04.87**

(51) Int. Cl.⁵: **A61K 35/16**

(54) **Steriles Plasmaaustauschmittel.**

(30) Priorität: **10.04.86 DE 3612137**

(43) Veröffentlichungstag der Anmeldung:
**25.11.87 Patentblatt 87/48**

(45) Bekanntmachung des Hinweises auf die Patenterteilung:
**05.09.90 Patentblatt 90/36**

(84) Benannte Vertragsstaaten:
**AT BE CH DE ES FR GB GR IT LI LU NL SE**

(56) Entgegenhaltungen:
**DE-A- 2 301 501**

(73) Patentinhaber: **Biotest Pharma GmbH,
Flughafenstrasse 4, D-6000 Frankfurt 71(DE)**

(72) Erfinder: **Kotitschke, Ronald, Dr. Dipl.-Chem.,
Kleiststrasse 23, D-6072 Dreieich(DE)**

(74) Vertreter: **Bell, Hans Chr., Dr., Bell, Wolff und Bell,
Rechtsanwälte Adelonstrasse 58 Postfach 80 01 40,
D-6230 Frankfurt am Main 80(DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft das in den Patentansprüchen beschriebene sterile Plasmaaustauschmittel.

Die therapeutische Plasmapherese (die Nomenklatur zur Plasmapherese und dem Plasmaaustausch ist in der Literatur leider nicht einheitlich; vorliegend wird unter der therapeutischen Plasmapherese oder dem Plasmaaustausch der Ersatz des Plasmas der Patienten durch ein anderes Substitutionsmittel verstanden) wird zur Elimination pathogener Substanzen aus dem Blut der Patienten verwendet. Die Indikationen für eine Austauschbehandlung können sehr unterschiedlich sein [(P.Reuther, D.Wiebecke, R.Rokkamm und H.G. Mertens: "Plasmaaustausch-Behandlungen bei neurologischen Krankheiten", Nervenarzt 54 (1983), 157-170 und H. Borberg, P.Reuther: "Plasma Exchange Therapy, Thieme, Stuttgart (1981)].

Neben der Entfernung der pathogenen Substanzen werden auch die normalen Plasmabestandteile entfernt. Das Ausmaß, mit dem die normalen Proteine entfernt werden und die Geschwindigkeit, mit der sie dem Körper wieder zurückggeben bzw. nachgebildet werden, ist ein entscheidendes Qualitätsmerkmal für das verwendete Austauschmittel.

Das Plasmaproteinprofil (darunter ist die Zusammensetzung der im Plasma vorkommenden Proteine und ihre Konzentration zu verstehen) Gesunder stellt ein körpereigenes Abwehrsystem dar, das in bezug auf die Konzentration der einzelnen Komponenten des Plasmas bestimmte Schwankungsbreiten zuläßt, die für die verschiedenen Proteine unterschiedlich sind und tageszeitlichen Schwankungsbreiten unterliegen [(A.Petralito, R.A.Mangiafico, S.Gibiino, M.A. Cuffari, M.F. Miano, C.E.Fiore: "Daily modifications of plasma fibrinogen, platelets aggregation, Howel's time, PTT, TT, and antithrombin III in normal subjects and in patients with vascular disease." Chronobiologia 9 (1982), 195-201].

Es ist das Kennzeichen vieler Krankheiten, daß das normal vorkommende Plasmaproteinprofil gestört ist und Abweichungen von der normalen Proteinzusammensetzung des Plasmas gravierende Folgen für den Organismus mit sich bringen können. Ist für die Behandlung bestimmter Krankheiten ein therapeutischer Plasmaaustausch indiziert, wäre es wünschenswert, das normale Plasmaproteinprofil durch die Substitution mit einem Mittel zu erhalten, das dazu in der Lage ist.

Die auf den Plasmaaustausch zurückzuführenden Veränderungen der Zusammensetzung der Proteine (Proteinprofil sind bei Verwendung kristalloider Lösungen und Albumin erheblich [J.B.Orlin und E.M. Berkman: "Partial plasma exchange using albumin replacement: removal and recovery of normal plasma constituents", Blood, Vol. 56, No. 6 (1980), 1055-1059; R.L. Volkin, T.W. Starz, A. Winkelstein, R.K. Shadduch, J.H. Lewis, U.Hasiba und J.A. Spero: "Changes in coagulation factors, complement, immunoglobulins, and immune complex concentrations with plasma exchange", Transfusion, Vol. 22, Nr. 1 (1982), 54-58; und M. Kuhlencordt, D.E.Vogel, C. Komm und A. Oberdorfer: "Veränderungen des plasmatischen Gerinnungssystems während Plasmapherese" Intensivmedizin 21 (1984) Nr. 6, 305-308].

Das heute am häufigsten verwendete Arzneimittel für den therapeutischen Plasmaaustausch ist die Albuminlösung mit einer Proteinkonzentration von 50 g/l. Die Verwendung des Albumins als Ersatz für alle anderen durch die therapeutische Plasmapherese entfernten Proteine ließ die Frage aufkommen, ob nicht die Abwesenheit der Immunglobuline eine erhöhte Produktion pathologisch wirksam werdender Immunglobuline in den plasmaausgetauschten Patienten provozieren könnte, da eine klassische Indikation für den therapeutischen Plasmaaustausch immunologisch bedingte Erkrankungen sind. Die Depletierung der Immunglobuline durch den Plasmaaustausch könnte auch das Risiko der Patienten erhöhen, an Infektionen zu erkranken. Zur Verminderung dieses Risikos ist daher frisch gefrorenes Plasma (FFP oder fresh frozen plasma) als Substitutionsmittel für den Plasmaaustausch verwendet worden. Fresh frozen plasma hatte aber eine sehr hohe Nebenwirkungsrate; bis zu 16 % der behandelten Patienten entwickelten Urticaria, bei einigen Patienten führten die Nebenwirkungen bis zum anaphylaktischen Schock. Die wiederholte Substitution mit FFP führt zu einer Kumulation der Fibrinogenkonzentration, und damit wegen der relativ langen Halbwertszeit des Fibrinogens zu einem erhöhten Thromboserisiko.

B. Stegmayr et al versuchten, das Problem der hohen Nebenwirkungsraten des FFP's dadurch zu vermeiden, daß sie anstelle des FFP's Plasma verwendeten, aus dem das Kryopräzipitat abgetrennt worden war [B. Stegmayr, B.Cedergren und B. Lindquist: Is stored liquid plasma or cryoprecipitate poor plasma an alternative for fresh frozen plasma as substitution in plasma exchange? Abstracts, International Symposium on Therapeutic Plasma Exchange and Selective Plasma Separation, Homburg/Saar (1985)].

Ein weiterer Grund für den Vorzug von Plasmaproteinfraktionen wie Albumin gegenüber Plasma als Austauschmedium beim therapeutischen Plasmaaustausch liegt in der limitierten Verfügbarkeit kompatibler Plasmen und dem Risiko der Hepatitisübertragung durch nicht sterilisierte Plasmen. Andererseits ist aber die Entfernung von drei bis fünf Litern Plasma bei einem Plasmaaustausch für eine dramatische Abnahme der Proteine mit biologischer Aktivität verantwortlich und kann unerwünschte Konsequenzen für das Hämostasepotential mit sich bringen. Wenn der Plasmaaustausch in zeitlich kurzen Abständen wiederholt wird, können sich die Nebenwirkungen addieren. Y.Sultan et al haben daher die Wirkungen des wiederholten Plasmaaustausches an Patienten mit Myasthenia gravis untersucht und nachgewiesen, daß bei der Verwendung von Albuminlösungen und Ringer-Lactat-Lösungen als Austauschmittel die behandelten Patienten der Gefahr eines erhöhten Thromboserisikos ausgesetzt werden [Y.Sultan, A.Bussel, P.Maisonneuve, M. Ponpeney, X.Sitty and P.Gajdos: "Potential Danger of Thrombosis after Plasma Exchange in the Treatment of Patients with Immune Disease", Transfusion: 19 (1979) 588-593].

Besondere Bedeutung bei der Auswahl eines optimalen Plasmaaustauschmittels kommt daher der folgenden Frage zu:

Gibt es ein Plasmaaustauschmittel mit Eigenschaften, die so sind, daß der Organismus in Folge des Austausches möglichst wenig von diesem Austausch beeinträchtigt wird und das normale Proteinprofil des Plasmas möglichst wenig verändert wird?

Theoretisch ist das ideale Austauschmedium für Blut Blut. Gegen die Verwendung von Vollblut für den Plasmaaustausch spricht dessen geringe Haltbarkeit. Für einen Plasmaaustausch ist es auch nicht notwendig, die Blutzellen des Patienten auszutauschen, da die Geräte und Maschinen, die für den therapeutischen Plasmaaustausch verwendet werden, so ausgestattet sind, daß dem Patienten seine eigenen Blutzellen zurückgeführt werden.

Bei der Stabilisierung von Blut und seiner Trennung in Zellen und das die Proteine enthaltende Plasma muß das Plasma unmittelbar nach seiner Gewinnung tiefgefroren werden, da einige der im Plasma enthaltenen Proteine sehr labil sind. Der Verwendung von FFP als Mittel der Wahl für den therapeutischen Plasmaaustausch stand in der Praxis vor allem die hohe Nebenwirkungsrate und das potentielle Risiko der Virusübertragung entgegen. Die Fraktionierung von Plasma in einzelne Proteinfraktionen erlaubt die Herstellung hochgereinigter Albuminlösungen, die nach Zugabe bestimmter Stabilisatoren pasteurisierbar sind. Eine Übertragung von Infektionskrankheiten läßt sich mit pasteurisierten Albuminlösungen vermeiden.

In der EP-A 0 014 333 ist eine mit β-Propiolacton und UV sterilisierte Serumproteinlösung mit einer Proteinkonzentration von 50 g/l beschrieben (Handelsname Biseko[R]), die in einer Studie, im Vergleich zu einer 5%igen Albuminlösung als Austauschmedium an fünf freiwilligen gesunden Probanden geprüft wurde [R.Kotitschke, H.Borberg, G.Güsken: "Langzeitstudie des Proteinprofils an gesunden Probanden nach einem Plasmaaustausch", In Abstracts and Handout-Poster - 29. Jahrestagung der DAB/GTH; Herausgeber: E.Wenzel und P.Hellstern, Homburg/Saar (1985), S.23].

Die 5%ige Serumproteinlösung führt nach dem Plasmaaustausch, im Vergleich zu der 5%igen Albuminlösung, zu einer deutlich geringeren Änderung der Konzentration einzelner Proteine, jedoch ist für einzelne Proteine diese Abweichung noch immer deutlich größer als die der normalen biologischen Schwankungsbreite.

Der Erfindung liegt die Aufgabe zugrunde, ein Mittel für den therapeutischen Plasmaaustausch zu finden, das das Risiko der Übertragung infektiöser Viren vermeidet und darüberhinaus als Folge seiner Anwendung das Plasmaproteinprofil der behandelten Patienten nahezu unverändert läßt.

Diese Aufgabe wird erfindungsgemäß durch ein steriles Plasmaaustauschmittel gelöst, das die humanen Serumproteine Albumin, die Immunglobuline IgG, IgA, IgM, die Inhibitoren Antithrombin III, alpha 1-Antitrypsin, alpha 2-Makroglobulin und den Komplementfaktor C3, außer den Gerinnungsfaktoren in einer Konzentration von 75 g/l enthält.

Gemäß einer besonders bevorzugten Ausführungsform enthält das erfindungsgemäße Plasmaaustauschmittel

35 bis 50 g/l Albumin,
6 bis 12 g/l IgG,
1,0 bis 2,5 g/l IgA,
0, 5 bis 2,5 g/l IgM,
10 bis 80 %d.N, Antithrombin III,
0,1 bis 0,3 g/l alpha 1-Antitrypsin,
0,1 bis 0,3 g/l alpha 2-Makroglobulin und
0,05 bis 0,15 g/l Komplementfaktor C3, wobei die Gesamtkonzentration der Serumproteine 75 g/l beträgt.

Das erfindungsgemäße sterile Plasmaaustauschmittel läßt sich beispielsweise durch Ankonzentrierung einer mit ß-Propiolacton und UV sterilisierten Serumproteinlösung herstellen, wie sie in der EP-A 0 014 333 beschrieben ist.

Die Erfindung beruht auf dem überraschenden Befund, daß eine gegenüber der bekannten 5%igen Serumproteinlösung um 25 g/l höhere Serumproteinkonzentration dazu führt, daß die Anwendung dieser Lösung beim therapeutischen Plasmaaustausch zu keiner nennenswerten Veränderung des Proteinprofils der behandelten Personen führt. Der Vergleich des Plasmaaustauschs an fünf freiwilligen gesunden Probanden mit einer Albuminlösung (Proteingehalt 50 g/l), der bekannten Serumproteinlösung (Proteingehalt 50 g/l) und der erfindungsgemäßen Serumproteinlösung (Proteingehalt 75 g/l) zeigt, daß durch die Höherkonzentrierung der Serumproteinlösung mit einem Proteingehalt von 50 g/l auf 75 g/l eine erstaunliche Täuschung des Organismus möglich ist. Der Organismus beantwortet den Plasmaaustausch mit der 5%igen Serumproteinlösung noch immer so, zwar in abgeschwächter Form, wie er es bei der Verwendung der 5%igen Albuminlösung getan hat, nämlich mit dem Versuch der Wiederherstellung der Ausgangskonzentration der Proteine durch eine Reaktion, die dazu führt, daß die Normalkonzentration nach dem Austausch über- oder unterschritten wird und die Wiederherstellung der Ausgangswerte Tage und Wochen in Anspruch nehmen kann. Überraschenderweise gelingt es, diese Reaktion des Organismus als Folge eines Plasmaaustausches bei der Verwendung des erfindungsgemäßen Plasmaaustauschmittels, d.h. der Serumproteinlösung mit einem Proteingehalt von 75 g/l zu vermeiden.

Die Erfindung basiert auf den überraschenden Ergebnissen einer Plasmaaustauschstudie an fünf freiwilligen gesunden Probanden, an denen ein 75%iger Plasmaaustausch mit einer Albuminlösung (5%) der bekannten Serumproteinlösung (5%) und dem erfindungsgemäßen Plasmaaustauschmittel, d.h. einer Serumproteinlösung mit einer Proteinkonzentration von 75 g/l, in einem zeitlichen Abstand von mehreren Monaten durchgeführt wurde. Zur Ermittlung der Kontrollwerte wurde den an dieser Studie beteiligten Probanden, im Abstand von jeweils fünf Tagen, über einen Zeitraum von 60 Tagen Serum bzw. Plasma ohne vorhergehenden Plasmaaustausch abgenommen.

Damit die Ergebnisse der Vergleichsuntersuchung der drei verschiedenen Austauschmittel bei dem therapeutischen Plasmaaustausch in übersichtlicher Form miteinander verglichen werden konnten, ist aus den einzelnen Meßergebnissen der Seren bzw. Plasmen der fünf Probanden pro Austauschgruppe ein Durchschnittswert gebildet worden. Auf diese Weise war es möglich, die Ergebnisse über ein bestimmtes Protein pro Gruppe in Form eines Kurvenzuges darzustellen.

In der Serie der Abb. I bis IV ist eine identische Form der Darstellung der Ergebnisse für die verschiedenen Proteine verwendet worden. Die Zeitachse über 60 Tage ist in unterschiedliche Zeitabschnitte aufgeteilt. Die Substitution des Albumins in der mit der Albuminlösung behandelten Gruppe wird durch den sofortigen Anstieg der Albuminkonzentration nach dem Austausch deutlich, während dieser Wert in der mit der 5%igen Serumproteinlösung behandelten Gruppe zunächst etwas absinkt. Die Infusion der erfindungsgemäßen 7,5%igen Serumproteinlösung führt nach der Infusion zu so moderaten Änderungen, daß diese Abweichungen sich nicht von den normalen Tagesschwankungen unterscheiden lassen (s. Abb. I).

Die in der 5%igen Proteinlösung enthaltenen Immunglobuline IgG, IgA und IgM und die Inhibitoren alpha 1-Antitrypsin, alpha 2-Makroglobulin und Antithrombin III führen nach dem Plasmaaustausch mit der 5%igen Serumproteinlösung dazu, daß die Konzentration dieser Proteine nicht so stark abfällt wie nach dem Albuminaustausch. Der Austausch mit der 5%igen Serumproteinlösung führt, in bezug auf die Wiederherstellung des normalen Plasmaproteinprofils, im Vergleich zu Albumin, zu weitaus geringeren Abweichungen von dem normalen Proteinprofil.

Der überraschende Befund der Plasmaaustauschstudie mit der 7,5%igen Serumproteinlösung besteht darin, daß eine Erhöhung der Proteinkonzentration von 50 g/l auf 75 g/l des Austauschmediums dazu führt, daß das normale Proteinprofil innerhalb kürzester Zeit nach dem Austausch wiederhergestellt ist. Aufgrund der Ergebnisse der Plasmaaustauschstudie mit der 5%igen Serumproteinlösung wäre zu erwarten gewesen, daß ein Austausch mit der gleichen Proteinlösung, bei der die Gesamtproteinkonzentration von 50 g/l auf 75 g/l ankonzentriert worden ist, zu dem vom Austausch mit der 5%igen Serumproteinlösung her bekannten Proteinprofil hätte führen müssen, da es sich um die gleiche Proteinlösung gehandelt hat. Da in beiden Lösungen, d.h. in der 5%igen Serumproteinlösung und der erfindungsgemäßen 7,5%igen Serumproteinlösung, exakt die gleichen Proteine vorliegen, hätte bereits die Infusion unterschiedlicher Mengen der 5%igen Serumproteinlösung zu unterschiedlichen Proteinprofilen führen müssen. Dies ist jedoch nicht der Fall gewesen. Überraschenderweise ist das nach einem Plasmaaustausch auftretende Proteinprofil also nicht nur von der Menge und der Zahl der mit dem Austauschmittel infundierten Proteine abhängig, sondern erfindungsgemäß in besonderem Maße von der Proteinkonzentration des Plasmaaustauschmittels (s. dazu die Ergebnisse der Vergleichsstudie in den Abb. I bis IV).

Der Vergleich des Verlaufs des Proteinprofils fur den Faktor VIII in Abhängigkeit von dem verwendeten Austauschmittel zeigt mehrere überraschende Ergebnisse. In der "Langzeitstudie des Proteinprofils an gesunden Probanden nach einem Plasmaaustausch" (s.oben) wurden bereits zwei als überraschend zu bewertende Beobachtungen mitgeteilt: Erstens der unterschiedliche Verlauf bei der Einstellung der Gerinnungsfaktoren VIII und IX auf ihre Normalwerte, in Abhängigkeit vom Austauschmedium, und zweitens die Zeitdauer dieses Vorganges nach dem Austausch mit Albumin.

Der Vergleich des Verlaufes der Wiederherstellung der normalen Proteinkonzentration, in Abhängigkeit vom Plasmaaustauschmedium zeigt, daß die Reaktion des Organismus bei dem Versuch, diesen Ausgleich zu erreichen, abhängig ist von dem Ausmaß der Abweichung vom Normalzustand. Ist die Abweichung sehr groß, wie beim Albuminaustausch, erfolgt eine heftige Reaktion, die zu den nach dem Albuminaustausch beobachteten dramatischen Konzentrationsänderungen für den Faktor VIII führt (s.Abb. IV). Da weder Albumin noch die 5%ige Serumproteinlösung Gerinnungsfaktoren enthalten, konnte, in bezug auf die Wiederherstellung der Ausgangskonzentration dieser Proteine, nach dem Austausch für diese beiden Austauschmedien ein übereinstimmender Reaktionsverlauf angenommen werden. Die tatsächlich gemessenen Werte für die Gerinnungsfaktoren I und VIII haben diese Annahme widerlegt. Als überraschend, im Vergleich zu den Verläufen der Wiederherstellung der Konzentration des Gerinnungsfaktors VIII, nach dem Austausch mit Albumin und der 5%igen Serumproteinlösung, ist dieser Verlauf nach dem Austausch mit der erfindungsgemäßen 7,5%igen Serumproteinlösung zu bewerten, da nach dem Plasmaaustausch mit dieser Lösung das Phänomen des Einpendelns der Konzentration dieses Proteins auf die Normalkonzentration durch zunächst ein Unterschreiten und anschließend ein Überschreiten der Normalkonzentration unterbleibt. Der Austausch mit dem erfindungsgemäßen Austauschmedium führt nur noch zu einem kurzfristigen Unterschreiten der Normalkonzentration nach dem Austausch, aber nicht mehr zu einem Überschreiten der Normalkonzentration. Der Verlauf der Annäherung der Konzentration des Faktors VIII an die Normalkonzentration entspricht überraschenderweise jetzt dem Verlauf, wie er für alle übrigen Proteine gefunden wurde. Die Besonderheiten, die für das Faktor VIII-Proteinprofil nach dem Austausch mit Albumin und der 5%igen Serumproteinlösung auftraten, entfallen nach dem Austausch mit der 7,5%igen Serumproteinlösung (s. Abb.IV).

Die Unterschiede zwischen den Proteinprofilen, die sich als Folge der verschiedenen Plasmaaustauschmittel analytisch feststellen lassen, so wie sie für einzelne Proteine in den Abb. I bis IV dargestellt wurden, zeigen sich eindrucksvoll bereits in einem makroskopischen Befund anhand der zu verschiedenen Zeitpunkten nach dem Plasmaaustausch abgenommenen Plasmaproben. Ein Teil der Plasmaproben, die nach dem Plasmaaustausch mit Albumin abgenommen wurden, waren geronnen. Die Anzahl der geronnenen Plasmaproben ist mit arabischen Zahlen angegeben, die zur Verdeutlichung an die Fibrinogenkurve der Abb. IV in den Kurvenzug geschrieben wurden. Diese Befunde bestätigen die zitierten Befunde über das Thromboserisiko der Patienten, die mit einem wiederholten Plasmaaustausch mit Albuminlösungen behandelt werden müssen. Zur Vermeidung des Thromboserisikos, als Folge eines Plasmaaustausches, ist ein ausgewogenes Hämostasepotential erforderlich, bei dem vor allem ein ausreichendes Inhibitorpotential dafür Sorge tragen muß, daß es zu keinem Übergewicht prokoagulatorischer Aktivitäten kommt. Besonders günstig für einen Plasmaaustausch sind also Mittel, mit denen eine ausreichende Menge an Inhibitoren zugeführt werden, um das potentielle Thromboserisiko zu vermeiden. Die Wirksamkeit der im erfindungsgemäßen Plasmaaustauschmittel enthaltenen Inhibitoren geht aus der Abb. III am Beispiel des alpha 1-Antitrypsins hervor.

Ein weiterer entscheidender Vorteil der erfindungsgemäßen 7,5%igen Serumproteinlösung als Plasmaaustauschmittel gegenüber der 5%igen Albuminlösung besteht darin, daß es zu keiner Depletierung der Immunglobuline durch den Plasmaaustausch kommt und somit das Risiko für die Patienten vermieden wird, an Infektionen zu erkranken. Dieser Vorteil trifft zwar auch auf die 5%ige Serumproteinlösung zu, jedoch ist aus der Abb. II zu erkennen daß die erfindungsgemäße 7,5%ige Serumproteinlösung gegenüber der 5%igen Serumproteinlösung quantitative Vorteile besitzt, da hier die IgG-Normalkonzentration bereits innerhalb eines Tages nach dem Plasmaaustausch wieder erreicht wird.

Die Herstellung eines erfindungsgemäßen Plasmaaustauschmittels wird durch das folgende Beispiel erläutert.

## Beispiel

Neun Teile Spender-Venenblut wurden zu einem Teil einer 3,8%igen Natriumcitrat-Stabilisatorlösung gegeben. Das Blut wurde möglichst bald nach der Blutentnahme zentrifugiert und die in physiologischer Kochsalzlösung aufgeschlemmten Erythrozyten dem Spender reinfundiert. Das Plasma wurde spätestens innerhalb von 48 Stunden bei -40°C eingefroren.

Das gefrorene Plasma wurde bei einer Temperatur von +2 bis +4°C aufgetaut. Das Kryopräzipitat wurde durch Zentrifugieren abgetrennt.

Der kryopräzipitatfreie Citratplasmapool wurde bei Raumtemperatur bis zu einer Konzentration von 0,25 Vol.-% mit frisch destilliertem β-Propiolacton versetzt. Es wurde eine Stunde bei Raumtemperatur gerührt und während dieser Zeit durch kontinuierliche Zugaben von 1N NaOH-Lösung der pH-Wert bei 7,2 gehalten. Nach UV-Bestrahlung mit einem UV-Durchfluß-Bestrahlungsgerät (Dill-Apparatur) wurde die Hydrolyse des β-Propiolactons durch kontinuierliche Zugabe von 1N NaOH unter Konstanthaltung des pH-Wertes zu Ende geführt, bis eine pH-Konstanz ohne weitere NaOH-Zugabe erreicht war. Das mit β-Propiolacton behandelte und UV-bestrahlte Citratplasma wurde auf 4°C abgekühlt und bei einem pH-Wert von 7,2 unter Rühren mit 0,5 g DEAE-Sephadex(R) A 50 (Diethylaminoethyl-Sephadex, Handelsnamen der Fa. Pharmacia Fine Chemicals für ein mit Epichlorhydrin quervernetztes Dextran) pro 1 Plasma adsorbiert.

Nach der Adsorption am DEAE-Sephadex A 50 wurde das Plasma vom Sephadex abgesaugt und diente als Ausgangsmaterial für die Herstellung der Lösung der Serumproteine.

Das nach der DEAE-Sephadex-Adsorption verbliebene Plasma wurde 3 Stunden bei +45°C mit 3% kolloidaler Kieselsäure (Aerosil AE 380 der Fa. Degussa) adsorbiert und anschließend zur Abtrennung der kolloidalen Kieselsäure zentrifugiert. Die erhaltene Lösung wurde zur Klärung über heißluftsterilisierte Membranen einer Porengröße von 5,0 μm gegeben.

Um den Gehalt der nach der Kieselsäure-Adsorption geklärten Lösung an Silikat, Calcium und Phosphat zu verringern, wurde sie unter Verwendung von Ultrafiltrationspatronen (Amicon PM 10) bei etwa +20°C ultrafiltriert. Die so erhaltene Lösung wurde auf eine Proteinkonzentration von 75 g/l ankonzentriert. Zur Einstellung der Elektrolyte wurden die entsprechenden Salze bis zu den gewünschten Konzentrationen zugegeben. Die ultrafiltrierte und ankonzentrierte, auf die gewünschte Elektrolytkonzentration eingestellte Proteinlösung wurde sodann über heißluftsterilisierte Membranfilter der Porengröße 0,45 und 0.22 μm in leere, sterile 1 l-Flaschen abgefüllt.

Die Zusamensetzung und die Eigenschaften der erhaltenen 7,5%igen Serumproteinlösung sind in der folgenden Tabelle zusammengestellt.

## TABELLE

### Zuammensetzung und Eigenschaften der 7,5%igen Serumproteinlösung

| | | | |
|---|---|---|---|
| Protein (g/l) | 75 | **Elektrolyte** | |
| Albumin (g/l) | 45–50 | Na (mäq/l) | 144–170 |
| IgG (g/l) | 9–10 | $K^{\oplus}$ (mäq/l) | 3,1–4,7 |
| IgA (g/l) | 1,7–2,0 | $Ca^{\oplus\oplus}$ (mäq/l) | 2,6–5,6 |
| IgM (g/l) | 0,7–1.0 | $Cl^{\ominus}$ (mäq/l) | 109–191 |
| AT III % d.N. | 25–40 | $Mg^{\oplus\oplus}$ (mäq/l) | 1,7–2,3 |
| $\alpha$1-Antitrypsin (g/l) | 0,2–0,25 | Citrat (mäq/l) | 15–25 |
| $\alpha$2-Makroglobulin (g/l) | 0,2–0,25 | Phosphat (mäq/l) | 1,0–2,0 |
| Komplementfaktor C3 (g/l) | 0,1–0,12 | Silikat (mäq/l) | < 15,0 |
| Cholinesterase (U/l) | ~ 200 | Hb (g/l) | < 0,05 |
| Ges.Lipide (g/l) | < 0,05 | Eisen (µg/dl) | 140–180 |
| Cholesterin (g/l) | < 0,05 | | |
| Glucose (g/l) | < 0,05 | PKA (% d.N.) | negativ |
| Celluloseacetatfolien-Elektrophorese: | | | |
| Albumin Rel. % | 60–70 | Anti-A(Titer) | $\leqq$ 1:64 |
| alpha 1-Proteine | 3–4 | Anti-B (Titer | $\leqq$ 1:64 |
| alpha 2-Proteine | 8–11 | HBsAg | negativ |
| beta-Proteine | 8–11 | KBR (Ch 50/mg | > 0,4 |
| gamma-Proteine | 11–16 | | |

EP 0 246 439 B1

| Bakt. Ak-Titer | | Virale Ak-Titer | |
|---|---|---|---|
| E.coli | 1:80 | Herpes 1 | 1:5 |
| Ps.aeruginosa | 1:160 | Herpes 2 | 1:5 |
| Klebs. | 1:40 | VZV | 1:5 |
| Staph. | 1:40 | CMV | 1:5 |
| Str.haem. | 1:40 | Röteln | 1:64 |
| Str.virid. | 1:20 | | |

**Patentansprüche für die Vertragsstaaten: BE, CH, DE, FR, GB, IT, LU, NL, SE**

1. Steriles Plasmaaustauschmittel, enthaltend die humanen Serumproteine Albumin, die Immunglobuline IgG, IgA, IgM, die Inhibitoren Antithrombin III, alpha 1-Antitrypsin, alpha 2-Makroglubilin und den Komplementfaktor C3 in einer Konzentration von 75 g/l.

2. Plasmaaustauschmittel nach Anspruch 1, dadurch gekennzeichnet, daß es
35 bis 50 g/l Albumin,
6 bis 12 g/l IgG,
1,0 bis 2,5 g/l IgA,
0,5 bis 2,5 g/l IgM,
10 bis 80% d.N. Antithrombin III,
0,1 bis 0,3 g/l alpha 1-Antitrypsin,
0,1 bis 0,3 g/l alpha 2-Makroglobulin und
0,05 bis 0,15 g/l Komplementfaktor C3
enthält, wobei die Gesamtkonzentration der Serumproteine 75 g/l beträgt.

**Patentansprüche für folgende Vertragsstaaten : AT, ES, GR**

1.Verfahren zur Herstellung eines sterilen Plasmaaustauschmittels, welches die humanen Serumproteine Albumin, die Immunglobuline IgG, IgA, IgM, die Inhibitoren Antithrombin III, alpha 1-Antitrypsin, alpha 2-Makroglobulin und den Komplementfaktor C3 enthält, dadurch gekennzeichnet, daß man eine aus Blutplasma durch Sterilisation und Entfernung von Fibrinogen, Citrat- und Calciumionen, Gerinnungsfaktoren II, VII, IX und X sowie von weiteren unstabilen Proteinen erhaltene Lösung der lagerstabilen Serumproteine auf eine Proteinkonzentration von 75 g/l einstellt.

2. Verfahren nach Anspruch 1, dadurch gekennzeichnet, daß man eine Lösung gewinnt, die
35 bis 50 g/l Albumin,
6 bis 12 g/l IgG,
1,0 bis 2,5 g/l IgA,
0,5 bis 2,5 g/l IgM,
10 bis 80% d.N. Antithrombin III,
0,1 bis 0,3 g/l alpha 1-Antitrypsin,
0,1 bis 0,3 g/l alpha 2-Makroglobulin und
0,05 bis 0,15 g/l Komplementfaktor C3 enthält, wobei die Gesamtkonzentration der Serumproteine 75 g/l beträgt.

**Claims for the Contracting States: BE, CH, DE, FR, GB, IT, LU, NL, SE**

1. Sterile plasma exchange medium containing the human serum proteins, albumin, the immunoglobulins IgG, IgA and IgM, the inhibitors antithrombin III, alpha 1-antitrypsin and alpha 2-macroglobulin and the complement factor C3, at a concentration of 75 g/l.

2. Plasma exchange medium according to Claim 1, characterised in that it contains from 35 to 50 g/l of albumin, from 6 to 12 g/l of IgG, from 1.0 to 2.5 g/l of IgA, from 0.5 to 2.5 g/l of IgM, from 10 to 80% of normal of antithrombin III, from 0.1 to 0.3 g/l of alpha 1-antitrypsin, from 0.1 to 0.3 g/l of alpha 2-macroglobulin and from 0.05 to 0.15 g/l of complement factor C3, the total concentration of serum proteins amounting to 75 g/l.

**Claims for the Contracting States: AT, ES, GR**

1. Process for the preparation of a sterile plasma exchange medium containing the human serum proteins, albumin, the immunoglobulins, IgG, IgA, and IgM, the inhibitors, antithrombin III, alpha 1-antitrypsin, and alpha 2-macroglobulin, and the complement factor C3, characterised in that a solution of storage stable serum proteins obtained from blood plasma by sterilization and removal of fibrinogen, citrate ions and calcium ions, clotting factors II, VII, IX and X and other unstable proteins is adjusted to a protein concentration of 75 g/l.

2. Process according to Claim 1, characterised in that a solution containing from 35 to 50 g/l of albumin, from 6 to 12 g/l of IgG, from 1.0 to 2.5 g/l of IgA, from 0.5 to 2.5 g/l of IgM, from 10 to 80% of normal of antithrombin III, from 0.1 to 0.3 g/l of alpha 1-antitrypsin, from 0.1 to 0.3 g/l of alpha 2-macroglobulin and from 0.05 to 0.15 g/l of complement factor C3 is obtained, the total concentration of serum proteins amounting to 75 g/l.

**Revendications pour les Etats contractants: BE, CH, DE, FR, GB, IT, LU, NL, SE**

1. Substitut de plasma stérile contenant les protéines sériques humaines albumine, les immunoglobulines IgG, IgA, IgM, les inhibiteurs antithrombine III, alpha 1-antitrypsine, alpha 2-macroglobuline et le facteur complément C3 à une concentration de 75 g/l.

2. Substitut de plasma selon la revendication 1, caractérisé en ce qu'il contient

35 à 50 g/l d'albumine,

6 à 12 g/l d'IgG,

1,0 à 2,5 g/l d'IgA,

0,5 à 2,5 g/l d'IgM,

10 à 80% d.N. antithrombine III,

0,1 à 0,3 g/l d'alpha 1-antitrypsine,

0,1 à 0,3 g/l d'alpha 2-macroglobuline et 0,05 à 0,15 g/l de facteur complément C3, où la concentration totale des protéines sériques s'élève à 75 g/l.

**Revendications pour les Etats contractants: AT, ES, GR**

1. Procédé de préparation d'un substitut de plasma stérile qui contient les protéines sériques humaines albumine, les immunoglobulines IgG, IgA, IgM, les inhibiteurs antithrombine III, alpha 1-antitrypsine, alpha 2-macroglobuline et le facteur complément C3, caractérisé en ce qu'on règle à une concentration de protéines de 75 g/l une solution des protéines sériques stables à la conservation obtenue à partir du plasma sanguin par stérilisation et enlèvement du fibrinogène, des ions citrate et calcium, des facteurs de coagulation II, VII, IX et X ainsi que d'autres protéines instables.

2. Procédé selon la revendication 1, caractérisé en ce qu'on obtient une solution qui contient

35 à 50 g/l d'albumine,

6 à 12 g/l d'IgG,

1,0 à 2,5 g/l d'IgA,

0,5 à 2,5 g/l d'IgM,

10 à 80% d.N. antithrombine III,

0,1 à 0,3 g/l d'alpha 1-antitrypsine,

0,1 à 0,3 g/l d'alpha 2-macroglobuline et

0,05 à 0,15 g/l de facteur complément C3 , où la concentration totale des protéines sériques s'élève à 75 g/l.

Abb. I

Albuminkonzentration in den Probandenplasmen nach dem Plasmaaustausch mit der

7.5 %igen Serumproteinlösung ————————

5.0 %igen Serumproteinlösung ————————

Albumin

Abb. II

## Abb. II

IgG- und IgA-Konzentration in Probandenplasmen nach dem Plasmaaustausch mit der

7.5 %igen Serumproteinlösung ——————————

5.0 %igen Serumproteinlösung —— —— ——

Albuminlösung - - - - - - - - - -

**Abb. III**

α1-Antitrypsinkonzentration in den Probandenplasmen nach dem Plasmaaustausch mit der

| | |
|---|---|
| 7.5 %igen Serumproteinlösung | ──────── |
| 5.0 %igen Serumproteinlösung | ── ── ── |
| Albuminlösung | ─ ─ ─ ─ ─ ─ |

Abb. IV

F. VIII-Aktivität und Fibrinogenkonzentration nach dem Plasmaaustausch mit der

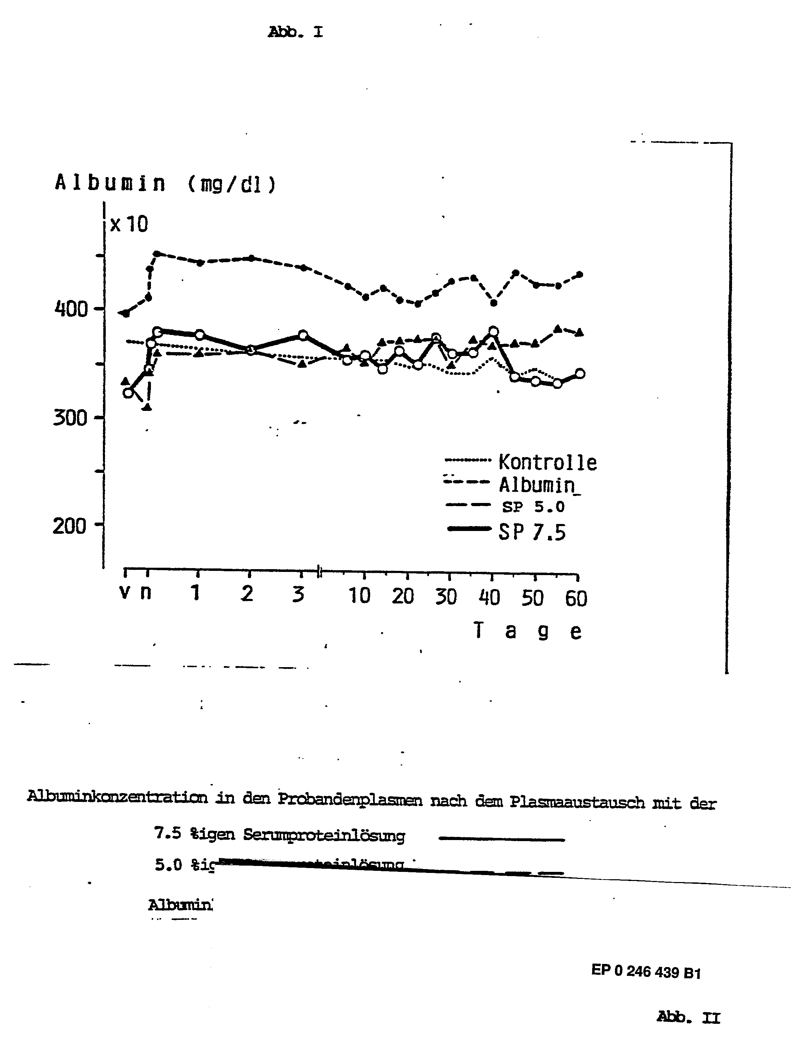

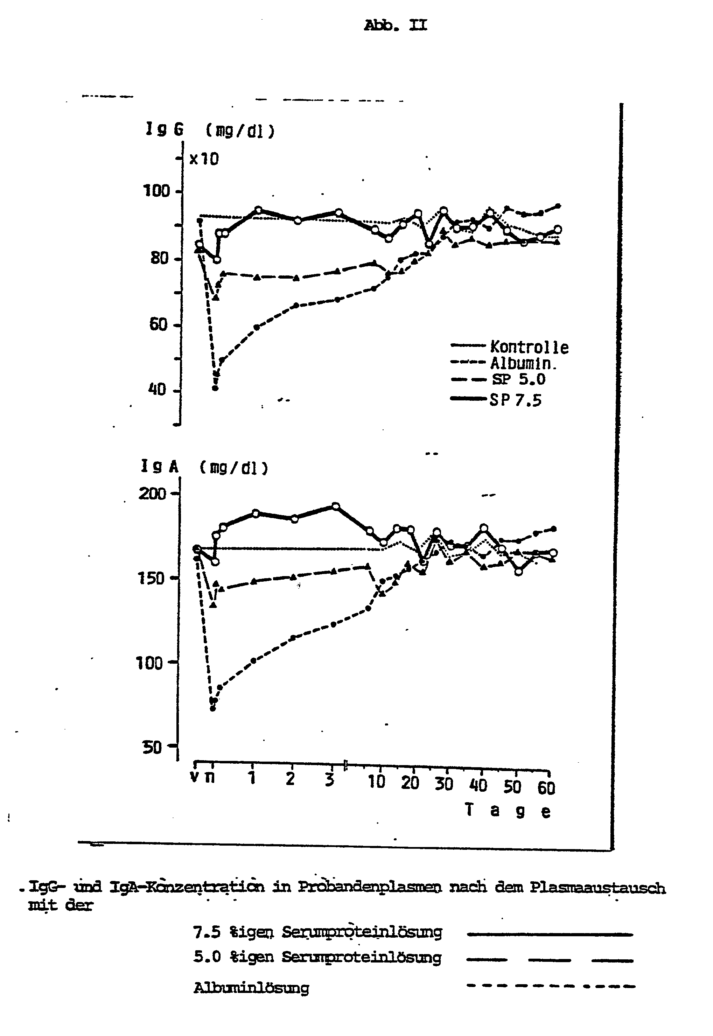

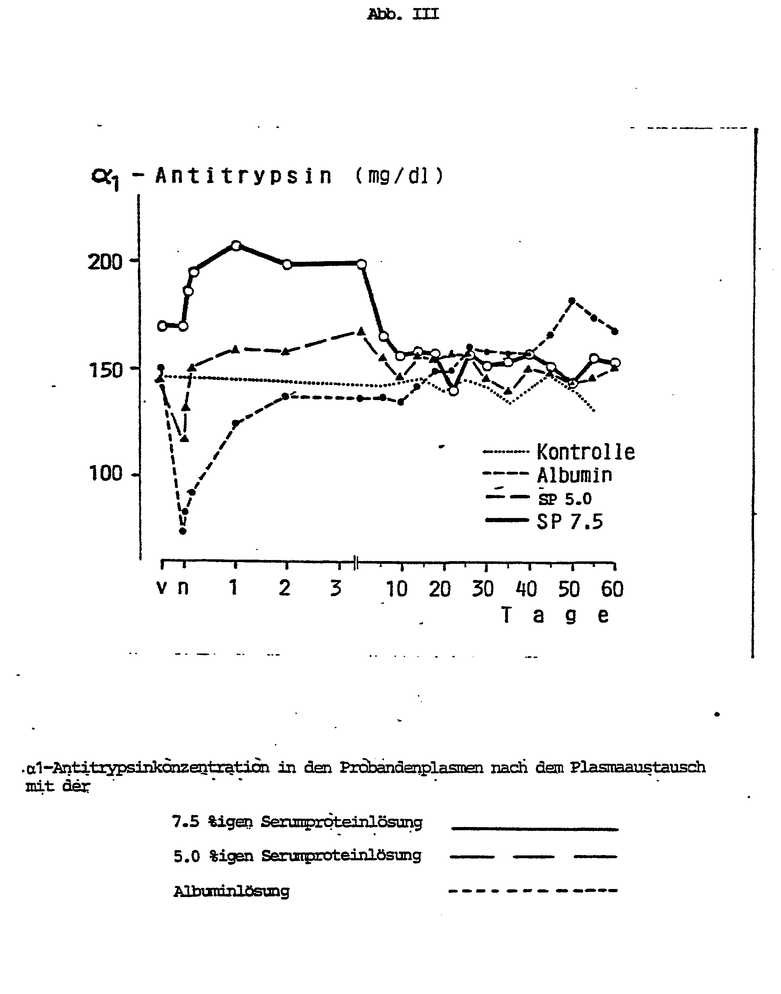

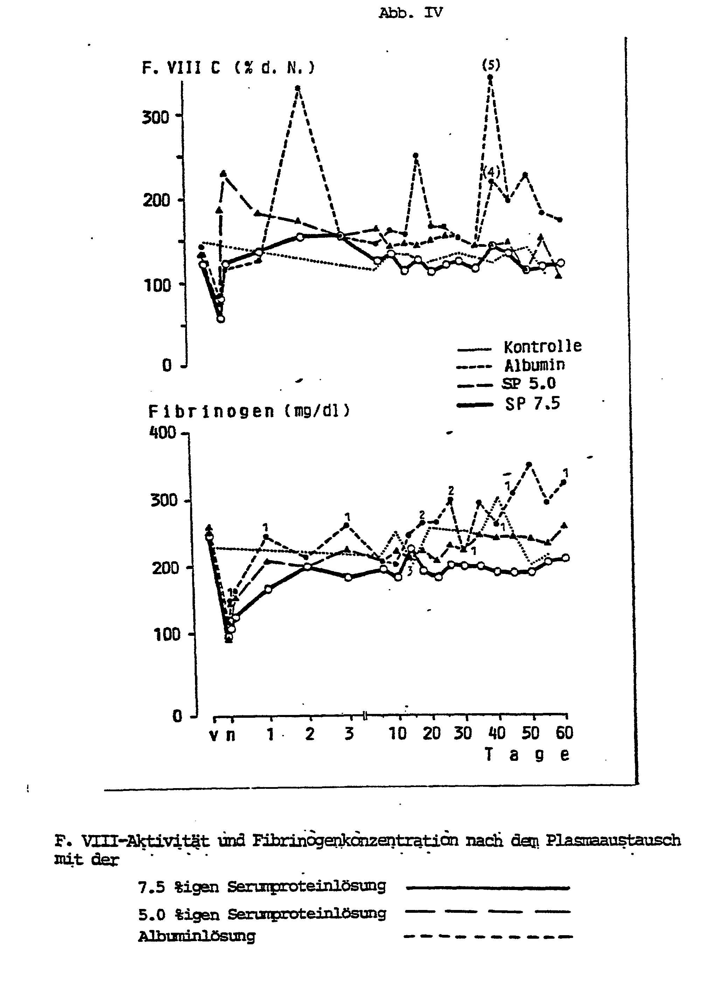

7.5 %igen Serumproteinlösung  ——————————

5.0 %igen Serumproteinlösung  —— —— —— ——

Albuminlösung  — — — — — — — —